# EUROPÄISCHE PATENTSCHRIFT

(11) **EP 1 407 728 B1**
(45) Veröffentlichungstag und Bekanntmachung des Hinweises auf die Patenterteilung: **14.06.2006**
(21) Anmeldenummer: 02022528.0
(22) Anmeldetag: 07.10.2002
(51) Int. Cl.: A61F 2/36

(54) **Femurkopfprothese**
Femoral head prosthesis
Prothèse de tête fémorale

(43) Veröffentlichungstag der Anmeldung: 14.04.2004
(73) Patentinhaber: Zimmer GmbH, 8404 Winterthur (CH)
(72) Erfinder: Prof. dr. med Spotorno, Lorenzo, 17024 Finale Ligure (IT); Willi, Roland, 8413 Neftenbach (CH); Grappiolo, Dr. Guido Maria, 17027 Finale Ligure (SV) (IT)
(74) Vertreter: Manitz, Finsterwald & Partner GbR

(56) Entgegenhaltungen:
- EP-A- 0 009 148
- EP-A- 0 094 829
- DE-C- 923 383
- FR-A- 1 017 927
- GB-A- 719 308
- US-A- 2 685 877
- US-A- 3 102 536
- US-A- 4 846 841
- US-A- 6 096 084

## Beschreibung

Die Erfindung handelt von einer Femurkopfprothese mit einem auf einem natürlichen Kugelstumpf in Richtung einer Halsachse A1 eines Femurknochens aufsetzbaren Kugelkopf mit Radius R und mit Mittelpunkt M, durch den eine Längsachse der Prothese gelegt ist, und mit einem Schaft der über eine untere Kante des Kugelkopfes zur Verankerung im Femurknochen vorsteht.

Eine derartige. Femurkopfprothese ist in der deutschen Patentschrift Nr. 923383 gezeigt. Die dort offenbarte Ausführung nimmt nur begrenzt auf die anatomischen Gegebenheiten im Femurkopf Rücksicht. Die Prothese ist als Zweistoffprothese aufgebaut, um mit einem körperverträglichen Kunststoff zum Knochenmaterial keine Reizerscheinungen oder Abstossungen zu provozieren. Eine dauerhafte Verankerung scheint mit dieser Prothese nicht möglich zu sein, da das Knochenmatieral im Grund der Kugelschale verödet und zu Abstossung führt. Eine Femurkopfprothese mit einer im Kugelkopf versenkten Gegenfläche für die vorderste Stirnfläche des Kugelstumpfes wird in US-A-6 096 084 beschrieben.

Die vorliegende Erfindung hat die Aufgabe eine Femurkopfprothese mit lang wirkender Verankerung zu schaffen. Diese Aufgabe wird mit dem Kennzeichen vom unabhängigen Anspruch 1 gelöst, indem die Prothese eine im Kugelkopf versenkte Gegenfläche für eine vorderste Stirnfläche des des Kugelstumpfes aufweist, welche vom Mittelpunkt M des Kugelkopfes aus gesehen zwischen den Grenzen 0.1 x Betrag des Radius R gegen distal und 0.2 x Betrag des Radius R gegen proximal angeordnet ist, um eine vorderste Stirnfläche des natürlichen Kugelkopfes als Abstützung zu verwenden die noch innerhalb des natürlichen Kugelkopfes aber gegen distal von einer Wachstumslinie liegt, welche durch eine Blutversorgung mit einer Arterie Ramus Acetabularis gegeben ist.

Eine solche Anordnung hat den Vorteil, dass zur Abstützung der Femurkopfprothese praktisch der grösstmögliche Teil an Knochenmaterial, welcher von distal her für seine Erneuerung mit Blut versorgt wird, verwendet werden kann. Im Gegensatz zu den Femurhalsprothesen, bei denen sich ein voller Kopf auf einem reserzierten Hals abstützt, werden die Belastungskräfte an einem reserzierten Kugelstumpf eingeleitet. Der Schaft ist nur so lang ausgeführt dass er im Rahmen der von der Kortikalis im Halsbereich zugelassenen Deformation zusätzlich Kräfte und Momente an die Spongiosa übertragen kann, ohne dass er selbst zu grosse Spannungen oder Deformationen wegen seiner grösseren Steifigkeit in der Spongiosa erzeugt.

Vorteilhafte Ergänzungen der Erfindung ergeben sich aus den abhängigen Ansprüche 2 bis 13.

Dadurch dass der Schaft sich gegen distal verjüngt und mit seinem distalen Ende von der Längsachse der Prothese in einem Winkel α > 15° weg gekrümmt ist, bleibt er bei einem Absinken der Prothese ohne Drehung verkeilt. Wenn die Gegenfläche in radialer Richtung zur Längsachse durch einen äusseren Radius R1 mit einem Betrag zwischen 90% und 70% vom Radius R und mit einem inneren Radius R2 mit einem Betrag zwischen 20% und 35% vom Radius R begrenzt ist, ergibt sich zur Abstützung am Kugelstumpf ein ausreichendes Polster aus Spongiosa, das mit Blut versorgt ist. Dies wird um so mehr erfüllt, wenn die Gegenfläche zwischen den Radien R1 und R2 grösser ist als die halbe Querschnittsfläche des Kugelkopfes.

Eine weitere vorteilhafte Massnahme besteht darin, die Femurkopfprothese zweiteilig mit einem aufsetzbaren Schaft zu gestalten. Zum einen können mit einer Grösse des Kugelkopfes verschieden grosse Schäfte verwendet werden und zum anderen können als Schaftmaterial ein ausgesprochen körperverträgliches Material wie zum Beispiel Titan oder Titanlegierungen verwendet werden. Wenn der Schaft an seiner Basis als Platte endet, kann auch ein Grossteil der Gegenfläche aus dem körperfreundlichen Material wie zum Beispiel Titan oder Titanlegierungen verwendet werden. Wenn der Schaft an seiner Basis als Platte endet, kann auch ein Grossteil der Gegenfläche aus dem körperfreundlichen Material bestehen, was das Einwachsen von Knochenmaterial fördert. Steife Verbindungen zwischen Schaft und Kugelkopf können durch eine zentrale Dehnschraube oder allgemein durch Schrauben gewährleistet werden.
Die Verbindung zwischen Schaft und Kugelkopf kann auch durch eine am Schaft angebrachte Spreizvorrichtung erreicht werden welche in eine hinterschnittene Bohrung im Kugelkopf eintaucht und durch eine Schraube im Schaft Haken, die mit Biegefedern den Schaft gegen proximal verlängern, in die Hinterscheidung einpressen.Eine solche Hinterschneidung ist auch in Materialien, welche Oxidkeramik oder Siliziumkarbid enthalten, herstellbar, sodass beispielsweise auch ein Kugelkopf aus Oxidkeramik mit einem metallischen Schaft gepaart werden kann. Eine derartige Spreizvorrichtung lässt sich auch so gestalten, dass die Schraube durch eine Öffnung im Kugelkopf mit einem Schraubendreher erreichbar ist um die Verankerung der Haken vorzunehmen. Dies gestattet es den Schaft für sich zu implantieren, ein Schnittwerkzeug für die Seitenflächen der reserzierten Stirnfläche am implantierten Schaft auszurichten und nachträglich den Kugelkopf am implantierten Schaft zu befestigen.

Im folgenden wird die Erfindung anhand von Ausführungsbeispielen beschrieben. Es zeigen:
- Fig. 1:: schematisch einen natürlichen Kugelkopf eines Oberschenkelknochens mit angedeuteter Wachstumslinie;
- Fig. 2:: schematisch einen Längsschnitt durch eine am Femur aufgesetzte erfindungsgemässe Fermurkopfprothese;
- Fig.3:: schematisch einen Längsschnitt durch eine Fermurkopfprothese analog zu Figur 2 mit einem in einen Femurkopf einsetzbaren Schaft;
- Fig.4:: schematisch die Fermurkopfprothese von Figur 3 mit mehreren Befestigungsschrauben für den einsetzbaren Schaft;
- Fig.5:: schematisch ein weiteres Ausführungsbeispiel analog zu Figur 2 mit einem in einen Femurkopf einsetzbaren Schaft;
- Fig.6:: schematisch ein weiteres Ausführungsbeispiel analog zu Figur 2 mit einem in einen Femurkopf einsetzbaren Schaft; und
- Fig. 7:: schematisch ein weiteres Ausführugnsbeispiel analog zu Figur 6 mit einem auch auf einen implantierten Schaft aufsetzbaren Kugelkopf.

In den nachfolgend beschriebenen Figuren sind gleiche Hinweiszeichen für gleiche Funktionen verwendet.

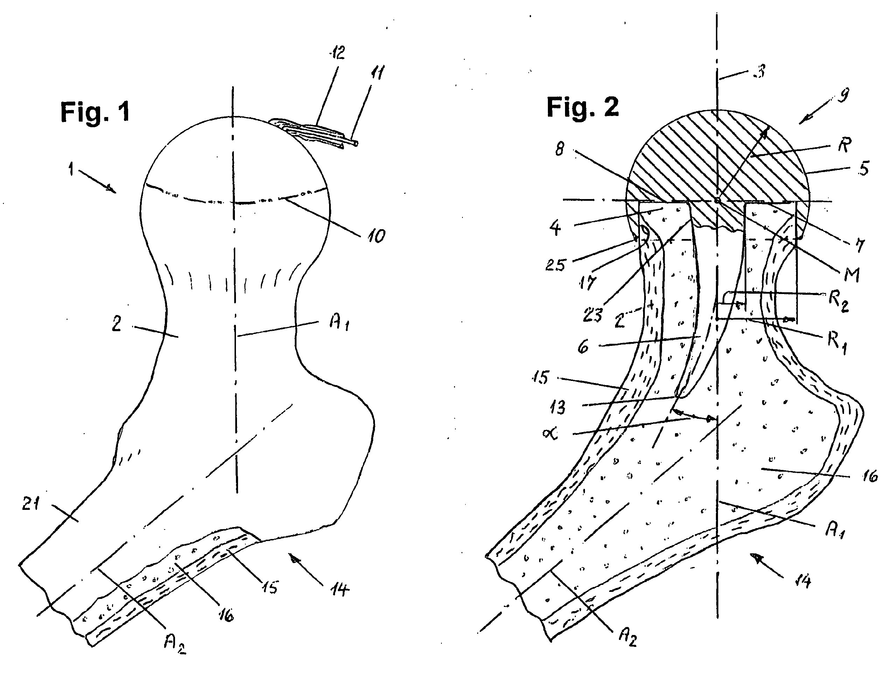

In der Figur 1 ist ein natürlicher Kugelkopf 1 mit seinem Übergang in einen Femurhals 2 gezeigt, dessen Halsachse A1 in einem Winkel von etwa 135° von der Fermurlängsachse A2 wegsteht. Ein Fermurknochen 14 ist aus einer harten Knochenrinde, der Kortikalis 15, und einer weicheren, innenliegenden Knochenschicht, der Spongiosa 16, aufgebaut. Auf dem natürlichen Kugelkopf 1 ist eine Wachstumslinie 10 angedeutet, die vor allem in jungen Jahren die Bedeutung hat, dass das an dieser Linie entstehende Knochengewebe von unterschiedlichen Seiten mit Blut versorgt wird. Der proximale, obere Teil wird durch die Arterie Ramus Acetabularis versorgt, welche am Ligament Capitis Femoris anliegt. Der untere distale Teil des natürlichen Kugelkopf wird durch Blutgefässe im Femurknochen versorgt. Auch bei älteren Menschen dient die Arterie Ramus Acetabularis immer noch einer beschränkten Blutversorgung.

Mit dem Öffnen des Gelenks und dem Herauslösen des Fermurkopfes aus dem Acetabulum muss zwangsläufig das Ligament Capitis Femoris und mit ihm die Arterie Ramus Acetabularis durchtrennt werden. Eine Resektionsfläche, die an der Wachstumslinie 10 liegt bietet zwei Vorteile. Zum einen wird sie auch nach der Operation ausreichend mit Blut aus dem Femurknochen versorgt um sich selbst zu erneuern. Zum anderen kann ein grösstmöglicher Kugelstumpf aus sich erneuerndem Knochenmaterial für die Abstützung einer Femurkopfprothese verwendet werden. Dadurch dass im Gegensatz zum vollständigen Ersatz des natürlichen Kugelkopfes ein Teil des Kugelkopfes erhalten bleibt, erfolgt die Einleitung der Belastungskräfte schon vor dem eigentlichen Femurhals, was der natürlichen Situation näher kommt.

Im Beispiel von Figur 2 ist eine erfindungsgemässe Femurkopfprothese 9 gezeigt. Eine Gegenfläche 7 für eine Stirnfläche 8 eines reserzierten Femurkopfes in Form eines Stumpfes 4 bildet eine Ebene durch den Mittelpunkt M eines Kugelkopfes 5. An den Kugelkopf 5 ist ein Schaft 6 angeformt, der eine innere Begrenzungsfläche 23 bildet, während eine äussere zylinderische Begrenzungsfläche 17 den Stumpf umschliesst. Die Gegenfläche 7 ist durch einen äusseren Radius R1 und einen inneren Radius R2 begrenzt. Mit einer solchen Anordnung ist die Kortikalis 15 beim Übergang in den Femurhals 2 von aussen gefasst und es verbleibt ein Ring aus Spongiosa 16, der in der Richtung der Halsachse A1 dämpfend wirkt. Der Schaft 6 der Prothese ragt über eine vordere Kante 25 des Kugelkopfes hinaus und in den Femurknochen 14 hinein, wobei er sich gegen distal verjüngt und mit seinem Ende 13 in einem Winkel α von 25° von der Längsachse 3 der Femurkopfprothese 9 weg gekrümmt ist. Die äussere Begrenzungsfläche 17 kann gegen aussen ganz leicht konisch geöffnet sein, beispielsweise mit einem halben Konuswinkel von 2°, um eine dauerhafte Pressung zu erzeugen. Zur Vorbereitung für den Schaft 6 wird eine Kavität in den Femurknochen geraspelt, welche ein leichtes Untermass gegenüber der Schaftgeometrie besitzt, um ebenfalls eine erhöhte Pressung bei der Primärverankerung zu erzeugen. Der Femurhals 2 kann sich entsprechend seiner Elastizität unter einer Gewichtskraft des Acetabulums immer noch etwas ausbiegen, wobei ihm der Schaft 6, welcher sich mit seinem Ende 13 in der Spongiosa abstützt, hilft, Biegemoment aufzunehmen und an den Femurknochen 14 abzugeben. Der Kugelradius R kann 38 bis 60mm betragen.

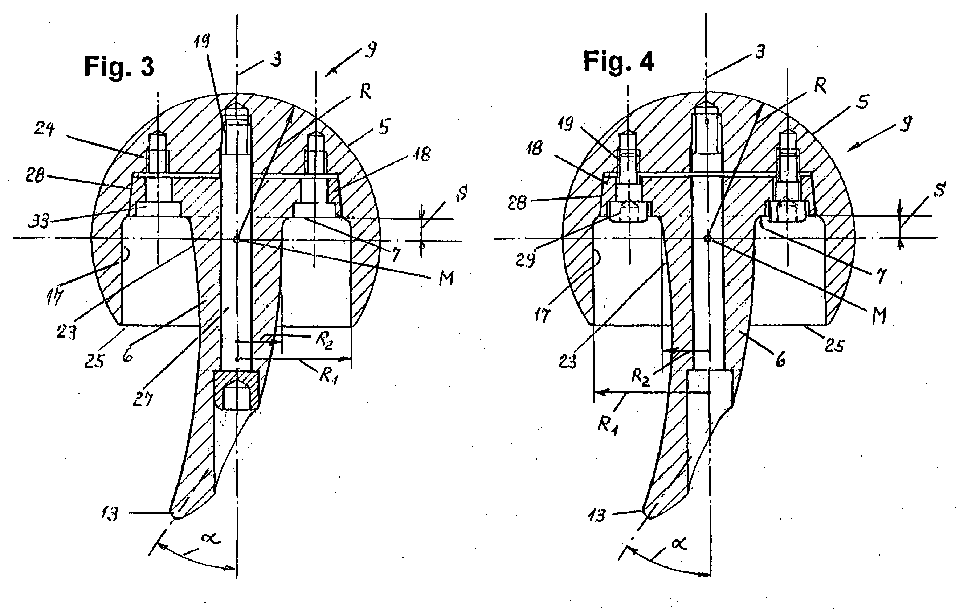

Im Beispiel von Figur 3 ist die Femurkopfprothese zweiteilig ausgeführt. In einen Kugelkopf 5 ist ein Schaft 6 einsetzbar, welcher an seiner Basis als Platte 18 endet. Die Platte 18 stützt sich über eine konische Klemmverbindung 28 am Kugelkopf 5 ab und wird über eine Schraubverbindung 19 mit einer Dehnschraube 27, welche in der Längsachse 3 verläuft, angepresst. Die Platte 18 ist Teil einer Gegenfläche 7, welche durch einen äusseren Radius R1 und einen inneren Radius R2 begrenzt ist. Die Gegenfläche 7 liegt zum Mittelpunkt M des Kugelkopfes 5 gegen proximal in einer Distanz S mit einem 0,15-fachen Betrag vom Radius R, um möglichst nahe bei der in Figur 1 gezeigten Wachstumslinie 10 zu liegen. Eine äussere Begrenzungsfläche 17 kann ebenfalls gegen aussen leicht konisch geöffnet sein. Ein Schaftende 13 steht gekrümmt mit einem Winkel α von 35° von der Längsachse 3 weg. In der Platte 18 sind weitere Bohrungen 33 und im Kugelkopf Gewinde 24 für Befestigungsschrauben (siehe Figur 4) angebracht. Der Schaft 6 kann aus einem körperverträglichen Material, beispielsweise Titan oder Titanlegierungen, bestehen, während der Kugelkopf 5 aus einem verschleissfesten Werkstoff, beispielsweise aus einer Chrom-Kobalt-Molybdänlegierung bestehen kann. Auf der eigentlichen Kugelfläche kann auch eine verschleissfeste nichtmetallische Schicht aus einem gesinterten Gefüge, beispielsweise aus Keramik oder polykristallinem Diamant aufgebacht sein.

Das Beispiel von Figur 4 entspricht dem von Figur 3 bis auf den Umstand, dass statt der zentralen Dehnschraube 27 mehrere Schrauben 29 die Platte 18 gegen den Kugelkopf 5 ziehen.

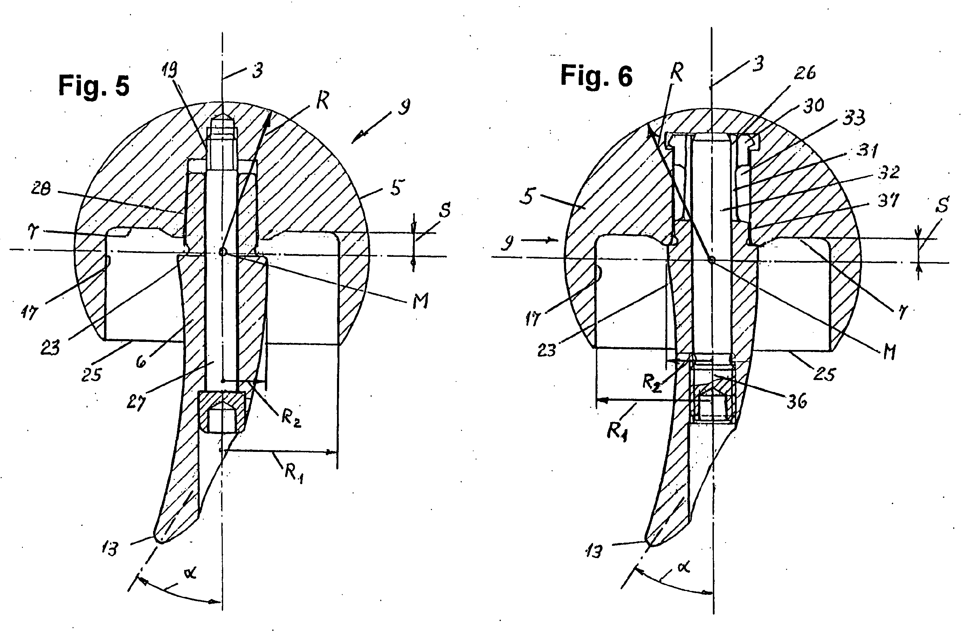

In Figur 5 ist ein weiteres Ausführungsbeispiel mit einem in einen Kugelkopf 5 einsetzbaren Schaft 6 gezeigt, der an seiner Basis durch einen konischen Stumpf fortgesetzt ist, um mit dem Kugelkopf 5 eine konische Klemmverbindung 28 zu bilden. Im Schaft 6 ist eine Dehnschraube 27 angeordnet, welche über eine Schraubverbindung19 in den Kugelkopf greift, um den Schaft festzuziehen. Eine proximal vom Mittelpunkt M gelegene Gegenfläche 7 hat eine Distanz S zum Mittelpunkt M des Kugelkopfes 5, die 15% vom Radius R ausmacht. In ihren äusseren Abmessungen und in ihren Materialien entspricht die Prothese dem Beispiel von Figur 3.

Ein weiteres Beispiel für eine Verbindung zwischen Kugelkopf 5 und Schaft 6 ist in Figur 6 gezeigt. Der Schaft 6 ragt in eine hinterschnittene Bohrung 33 des Kugelkopfes hinein und verankert sich dort mit einer Spreizvorrichtung 26. An seinem proximalen Ende zentriert sich der Schaft 6 in einer Bohrung 33 mit einer zylindrischen Schulter und ist dann weiter gegen proximal in Biegefedern 31 aufgeteilt, die in radialer Richtung nachgiebig sind und die in Haken 30 enden. Biegefedern 31 und Haken 30 sind durch einen zentralen Stempel 32 von innen radial abgestützt. Zur Montage des Schaftes 6 im Kugelkopf 5 wird der Schaft mit den Haken 30 und Biegefedern 31 in die Bohrung 33 eingeführt, wobei die Biegefedern und Haken soviel seitlichen Abstand zueinander haben, dass sie nach innen zurückfedern können. Erst wenn die Haken 30 den hinterschnittenen Teil der Bohrung 33 erreichen, können sie annähernd ihre normale Position einnehmen. Eine Schraube 36, deren Vorderteil als Stempel 32 verlängert ist, wird eingedreht bis der Stempel 32 die Haken 30 in ihrer Verriegelungspostition von innen stützt und stirnseitig anschlägt. Die äusseren Abmessungen und die Materialien entsprechen dem Beispiel von Figur 5.

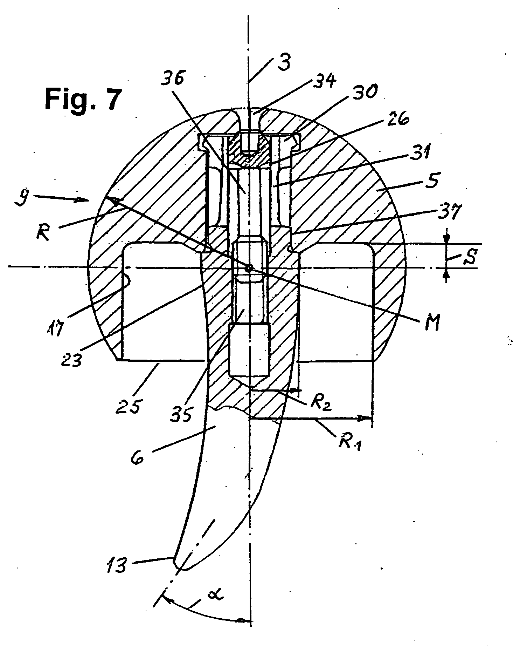

Den Beispielen von Figur 3 bis Figur 6 ist gemeinsam, dass der Schaft 6 am Kugelkopf 5 befestigt werden muss, bevor die Femurkopfprothese implantiert werden kann. In einem weiteren Ausführungsbeispiel gemäss Figur 7 ist ein Schaft 6 mit einer Spreizvorrichtung 26 ähnlich zum Beispiel der Figur 6 gezeigt, wobei die Spreizvorrichtung jedoch durch eine Öffnung 34 des Kugelkopfes 5 betätigbar ist. Der Schaft besitzt einen zylindrischen Ansatz 37 und eine als Sackloch von proximal ausgeführte Gewindebohrung 35, die es erlauben eine Setzvorrichtung (hier nicht gezeigt) anzubringen und den Schaft für sich in einem Kugelstumpf zu implantieren, der lediglich an seiner Stirnfläche reserziert ist. Nach dem entfernen der Setzvorrichtung kann an dem Ansatz 37 ein Schnittwerkzeug zentriert und ausgerichtet werden, welches den Kugelstumpf 4 an seiner Mantelfläche fertig bearbeitet. Anschliessend kann der eigentliche Kugelkopf 5 aufgesetzt und mit der Spreizvorrichtung 26 am Schaft 6 fixiert werden. Vor dem Aufsetzen des Kugelkopfes 5 wird eine Schraube 36 so tief in den Schaft 5 eingeschraubt, dass Biegefedern 31 und Haken 30 frei beweglich sind. Mit dem Aufsetzen des Kugelkopfes federn die Haken 30 nach innen und zurück in den hinterchnittenen Teil der Bohrung 36 im Kugelkopf. Der Kopf der Schraube 36 wird anschliessend gegen proximal gedreht bis er am Kugelkopf anschlägt. Er übernimmt die Funktion des Stempels 32 von Figur 6, indem er die Haken 30 radial verkeilt und in axialer Richtung verspannt.

### Bezeichnungen

- 1.: natürlicher Kugelkopf
- 2.: Femurhals
- 3.: Längsachse
- 4.: (Kugel)stumpf
- 5.: Kugelkopf
- 6.: Schaft
- 7.: Gegenfläche
- 8.: Stirnfläche
- 9.: Femurkopfprothese
- 10.: Wachstumslinie
- 11.: Arterie Ramus Acetabulis
- 12.: Ligament Capitis Femoris
- 13.: Ende
- 14.: Femurknochen
- 15.: Kortikalis
- 16.: Spongiosa
- 17.: äussere Begrenzungsfläche
- 18.: Platte
- 19.: Schraubverbindung
- 20.: Schraube
- 21.: Femurschaft
- 22.: oberer Kopfteil
- 23.: innere Begrenzungsfläche
- 24.: Gewinde
- 25.: vordere Kante
- 26.: Spreizvorrichtung
- 27.: Dehnschraube
- 28.: konische Klemmverbindung
- 29.: Schraube
- 30.: Haken
- 31.: Biegefeder
- 32.: Stempel
- 33.: Bohrung
- 34.: Öffnung
- 35.: Gewindebohrung
- 36.: Schraube
- 37.: zylindrischer Ansatz

- A1: Halsachse
- A2: Femurlängsachse
- M: Mittelpunkt
- R: Radius
- R1: äusserer Radius
- R2: innerer Radius
- S: Distanz
- α: Krümmungswinkel

## Patentansprüche

1. Femurkopfprothese mit einem auf einem natürlichen Kugelstumpf (4) in Richtung einer Halsachse A1 eines Femurknochens (14) aufsetzbaren Kugelkopf (5) mit Radius R und mit Mittelpunkt M, durch den eine Längsachse (3) der Prothese gelegt ist, und mit einem Schaft (6) der über eine untere Kante (25) des Kugelkopfes zur Verankerung im Femurknochen vorsteht, wobei die Prothese eine im Kugelkopf versenkte Gegenfläche (7) für eine vorderste Stirnfläche (8) des Kugelstumpfes aufweist, **dadurch gekennzeichnet, dass** die Gegenfläche vom Mittelpunkt M des Kugelkopfes aus gesehen zwischen den Grenzen 0,1 × Betrag des Radius R gegen distal und 0.2 × Betrag des Radius R gegen proximal angeordnet ist, um eine vorderste Stirnfläche (8) des natürlichen Kugelkopfes (4) als Abstützung zu verwenden, die noch innerhalb des natürlichen Kugelkopfes (4) aber gegen distal von einer Wachstumslinie (10) liegt, welche durch eine Blutversorgung mit einer Arterie Ramus Acetabularis gegeben ist.

2. Femurkopfprothese nach Anspruch 1 **dadurch gekennzeichnet, dass** der Schaft (6) sich gegen distal verjüngt und mit seinem distalen Ende (13) von der Längsachse (3) der Prothese mit einem Krümmungswinkel von α > 15° weg gekrümmt ist.

3. Femurkopfprothese nach einem der Ansprüche 1 oder 2, **dadurch gekennzeichnet, dass** die Gegenfläche (7) eben ausgeführt ist.

4. Femurkopfprothese nach einem der Ansprüche 1 bis 3, **dadurch gekennzeichnet, dass** die Gegenfläche (7) in radialer Richtung zur Längsachse (3) durch einen äusseren Radius R1 mit einem Betrag zwischen 90% und 70% vom Radius R und mit einem inneren Radius R2 mit einem Betrag zwischen 20% und 35% vom Radius R begrenzt ist.

5. Femurkopfprothese nach Anspruch 4, **dadurch gekennzeichnet, dass** die Fläche zwischen den Radien R1 und R2 grösser ist als die halbe Querschnittsfläche des Kugelkopfes (5) mit Radius R.

6. Femurkopfprothese nach einem der Ansprüche 1 bis 5, **dadurch gekennzeichnet, dass** die Prothese zweiteilig mit einem im Kugelkopf (5) aufsteckbaren Schaft (6) ausgeführt ist.

7. Femurkopfprothese nach Anspruch 6, **dadurch gekennzeichnet**, **das** der Schaft (6) aus einem körperverträglichen Material beispielsweise aus Titan oder einer Titanlegierung ausgeführt ist.

8. Femurkopfprothese nach Anspruch 6, **dadurch gekennzeichnet, dass** der Schaft (6) an seiner Basis als Platte (18) endet, welche am Kopf (5) über eine Schraubverbindung (19) befestigbar ist.

9. Femurkopfprothese nach Anspruch 6, **dadurch gekennzeichnet, dass** der Schaft (6) über eine konische Klemmverbindung (28) am Kopf (5) aufliegt.

10. Femurkopfprothese nach Anspruch 6, **dadurch gekennzeichnet, dass** der Schaft (6) zu seiner Verankerung im Kopf (5) eine Spreizvorrichtung (26) aufweist.

11. Femurkopfprothese nach Anspruch 10, **dadurch gekennzeichnet, dass** die Spreizvorrichtung (26) durch eine Öffnung (34) im Kugelkopf (5) betätigbar ist, damit der Kugelkopf (5) auch auf einem bereits implantierten Schaft (6) befestigbar ist.

12. Femurkopfprothese nach einem der Ansprüche 1 bis 11, **dadurch gekennzeichnet, dass** der Kugelkopf (5) aus einem körperverträglichen und verschleissfesten Material beispielsweise aus einer Chrom- Kobalt- MolybdänLegierung besteht.

13. Femurkopfprothese nach einem der Ansprüche 10 oder 11, **dadurch gekennzeichnet, dass** der Kogelkopf (5) ein körperverträgliches, nichtmetallisches Material wie beispielsweise Oxidkeramik oder Siliziumkarbid enthält.

## Claims

1. A femoral head prosthesis having a spherical head (5) which can be placed on a natural spherical stump (4) in the direction of a neck axis A 1 of a femur (14) and has a radius R and a centre M through which a longitudinal axis (3) of the prosthesis is laid, and having a shaft (6) which projects beyond a lower edge (25) of the spherical head for anchoring in the femur, wherein the prosthesis has a mating surface (7) countersunk in the spherical head for a foremost end face (8) of the spherical stump, **characterised in that** the mating surface is arranged between the boundaries 0.1 × amount of the radius R towards distal and 0.2 × amount of the radius R towards proximal, when viewed from the centre M of the spherical head, to use a foremost end surface (8) of the natural spherical head (4) as a support which still lies within the natural spherical head (4), but towards distal from a growth line (10) which is present due to a blood supply with an artery ramus acetabularis.

2. A femoral head prosthesis in accordance with claim 1, **characterised in that** the shaft (6) tapers towards distal and its distal end (13) curves away from the longitudinal axis (3) of the prosthesis at an angle of curvature of α > 15°.

3. A femoral head prosthesis in accordance with any one of the claims 1 or 2, **characterised in that** the mating surface (7) is planar.

4. A femoral head prosthesis in accordance with any one of the claims 1 to 3, **characterised in that** the mating surface (7) is bounded towards the longitudinal axis (3) in the radial direction by an outer radius R1 with an amount between 90% and 70% of the radius R and with an inner radius R2 with an amount between 20% and 35% of the radius R.

5. A femoral head prosthesis in accordance with claim 4, **characterised in that** the area between the radii R1 and R2 is larger than half the cross-sectional area of the spherical head (5) with a radius R.

6. A femoral head prosthesis in accordance with any one of the claims 1 to 5, **characterised in that** the prosthesis is made in two parts with a shaft (6) which can be placed in the spherical head (5).

7. A femoral head prosthesis in accordance with claim 6, **characterised in that** the shaft (6) is made of a biocompatible material, for example of titanium or of a titanium alloy.

8. A femoral head prosthesis in accordance with claim 6, **characterised in that** the shaft (6) ends at its base as a plate (18) which can be fastened to the head (5) via a screw connection (19).

9. A femoral head prosthesis in accordance with claim 6, **characterised in that** the shaft (6) lies on the head (5) via a conical clamping connection (28).

10. A femoral head prosthesis in accordance with claim 6, **characterised in that** the shaft (6) has a spreading device (26) for its anchoring in the head (5).

11. A femoral head prosthesis in accordance with claim 10, **characterised in that** the spreading device (26) can be actuated through an opening (34) in the spherical head (5) so that the spherical head (5) can also be fastened to a shaft (6) already implanted.

12. A femoral head prosthesis in accordance with any one of the claims 1 to 11, **characterised in that** the spherical head (5) consists of a biocompatible and wear-resistant material, for example of a chromium, cobalt, molybdenum alloy.

13. A femoral head prosthesis in accordance with any one of the claims 10 or 11, **characterised in that** the spherical head (5) contains a biocompatible, non-metallic material such as an oxide ceramic or silicon carbide.

## Revendications

1. Prothèse de tête fémorale, comprenant une tête sphérique (5) de rayon R et de centre M, susceptible d'être posée sur un moignon sphérique naturel (4) en direction d'un axe A1 du col d'un fémur (14), tête à travers laquelle est disposé un axe longitudinal (3) de la prothèse, et comprenant un tronc (6) qui dépasse au-delà d'une arête inférieure (25) de la tête sphérique pour l'ancrage dans l'os du fémur, dans laquelle la prothèse comporte une contre-surface (7) en renfoncement dans la tête sphérique pour une surface frontale (8) tout à fait antérieure du moignon sphérique, **caractérisée en ce que** la contre-surface est agencée, lorsqu'on la regarde depuis le centre M de la tête sphérique, entre les limites définies par 0,1 fois la valeur du rayon R en direction distale et 0,2 fois la valeur du rayon R en direction proximale, afin d'utiliser comme soutien une surface frontale (8) tout à fait antérieure de la tête sphérique naturelle (4), mais qui est disposée encore à l'intérieur de la tête sphérique naturelle (4) en direction distale depuis une ligne de croissance (10) qui est définie par une alimentation sanguine au moyen d'une artère Ramus Acetabularis.

2. Prothèse de tête fémorale selon la revendication 1, **caractérisée en ce que** le tronc (6) va en se rétrécissant en direction distale et est incurvé avec son extrémité distale (13) en éloignement de l'axe longitudinal (3) de la prothèse avec un angle de courbure α > 15°.

3. Prothèse de tête fémorale selon l'une des revendications 1 ou 2, **caractérisée en ce que** la contre-surface (7) est réalisée plane.

4. Prothèse de tête fémorale selon l'une des revendications 1 à 3, **caractérisée en ce que** la contre-surface (7) est limitée, en direction radiale par rapport à l'axe longitudinal (3), par un rayon extérieur R1 d'une valeur entre 90 % et 70 % du rayon R, et par un rayon intérieur R2 d'une valeur entre 20 % et 35 % du rayon R.

5. Prothèse de tête fémorale selon la revendication 4, **caractérisée en ce que** la surface entre les rayons R1 et R2 est supérieure à la moitié de la surface de section de la tête sphérique (5) de rayon R.

6. Prothèse de tête fémorale selon l'une des revendications 1 à 5, **caractérisée en ce que** la prothèse est réalisée en deux parties avec un tronc (6) susceptible d'être enfiché dans la tête sphérique (5).

7. Prothèse de tête fémorale selon la revendication 6, **caractérisée en ce que** le tronc (6) est réalisé en un matériau compatible avec le corps, par exemple en titane ou en alliage de titane.

8. Prothèse de tête fémorale selon la revendication 6, **caractérisée en ce que** le tronc (6) se termine à sa base comme une plaque (18), laquelle est susceptible d'être fixée sur la tête (5) via une liaison vissée (19).

9. Prothèse de tête fémorale selon la revendication 6, **caractérisée en ce que** le tronc (6) est appliqué sur la tête (5) via une liaison à coincement conique (28).

10. Prothèse de tête fémorale selon la revendication 6, **caractérisée en ce que** le tronc (6) présente un dispositif à écartement (26) pour son ancrage dans la tête (5).

11. Prothèse de tête fémorale selon la revendication 10, **caractérisée en ce que** le dispositif d'écartement (26) est susceptible d'être actionné à travers une ouverture (34) dans la tête sphérique (5), afin de pouvoir fixer la tête sphérique (5) également sur un tronc déjà implanté (6).

12. Prothèse de tête fémorale selon l'une des revendications 1 à 11, **caractérisée en ce que** la tête sphérique (5) est réalisée en un matériau compatible avec le corps et résistant à l'usure, par exemple un alliage chrome-cobalt-molybdène.

13. Prothèse de tête fémorale selon l'une des revendications 10 ou 11, **caractérisée en ce que** la tête sphérique (5) contient un matériau non métallique compatible avec le corps, comme par exemple une céramique à base d'oxyde ou du carbure de silicium.
